(19) Europäisches Patentamt
European Patent Office
Office européen des brevets

(11) Veröffentlichungsnummer: **0 378 767 B1**

# (12) EUROPÄISCHE PATENTSCHRIFT

(45) Veröffentlichungstag der Patentschrift: **31.08.94**

(51) Int. Cl.5: **C07C 51/58**, C07C 59/135

(21) Anmeldenummer: **89120269.9**

(22) Anmeldetag: **02.11.89**

Die Akte enthält technische Angaben, die nach dem Eingang der Anmeldung eingereicht wurden und die nicht in dieser Patentschrift enthalten sind.

## (54) Verfahren zur Oligomerisation von Hexafluorpropenoxid.

(30) Priorität: **14.01.89 DE 3901000**

(43) Veröffentlichungstag der Anmeldung:
**25.07.90 Patentblatt 90/30**

(45) Bekanntmachung des Hinweises auf die Patenterteilung:
**31.08.94 Patentblatt 94/35**

(84) Benannte Vertragsstaaten:
**BE CH DE ES FR GB IT LI NL**

(56) Entgegenhaltungen:
**EP-A- 0 203 466**
**DE-A- 2 627 986**
**GB-A- 1 550 268**

**PATENT ABSTRACTS OF JAPAN, Band 12, Nr. 22 (C-470)(2869), 22. Januar 1988; & JP-A-62 175 497**

**PATENT ABSTRACTS OF JAPAN, Band 12, Nr. 47 (C-475)(2894), 12. Februar 1988; & JP-A-62 195 345**

(73) Patentinhaber: **HOECHST AKTIENGESELLSCHAFT**

**D-65926 Frankfurt (DE)**

(72) Erfinder: **Strutz, Heinz, Dr.**
**Johannesallee 14**
**D-6230 Frankfurt am Main 80 (DE)**

EP 0 378 767 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Oligomeren des Hexafluorpropenoxides.

Die katalysierte Oligomerisation von Hexafluorpropenoxid (HFPO) ist bekannt; die dabei entstehenden Säurefluoride (I) weisen normalerweise eine breite Molmassenverteilung (n = 0 - 30) auf (Angew. Chem. (1985) 97, 164|.

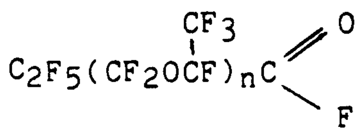

n= 0, 1, 2, ... (I)

Es sind nur wenige Beispiele für die selektive Dimerisierung von HFPO bekannt: ein System $AgNO_3$/Lösemittel führt zwar mit einer Selektivität von 86 % zum HFPO-Dimeren ((I) mit n = 1), ist aber - wie die meisten Silberverbindungen -lichtempfindlich und entwickelt problematisch einzustufende nitrose Gase (DE-A 20 26 669). Das System CuCl/$CuCl_2$/Acrylnitril führt ebenfalls mit guter Selektivität zum dimeren HFPO, ist aber durch das toxische Acrylnitril stark belastet (DE-A 29 24 385). Darüberhinaus wird der Chloranteil des Katalysators unerwünschterweise in das Produkt getragen. Weiterhin werden hohe Anforderungen an eine konstante Temperatur während der Reaktion gestellt.

Bei den literaturbekannten Katalysator-Systemen für die HFPO-Oligomerisation ist von einer wasserfreien Handhabung auszugehen. Nach einer weiteren Veröffentlichung werden gängige Oligomerisierungskatalysatoren durch Zusatz protonenaktiver Verbindungen zu selektiven Dimerisierungs-Katalysatoren (JP-A 62-1953459: Das in den Beispielen ausschließlich verwendete Cäsiumfluorid stellt allerdings ein schwer handhabbares (Hygroskopie) und - insbesondere auch mit Blick auf den teilweisen Austrag in das Oligomergemisch - sehr teures Katalysatorsystem dar. Nach den angeführten Beispielen ist weiterhin anzunehmen, daß die Einhaltung einer Reaktionstemperatur von minus 20 °C für das Reaktionsresultat unbedingt erforderlich ist, eine solche Reaktionstemperatur ist jedoch für eine technische Anwendung nachteilig.

Der Einsatz des in JP-A 62-195345 ebenfalls beanspruchten Systems tert.-Amin/protonenaktive Verbindung als selektiver Dimerisierungskatalysator konnte unter den angegebenen, insbesondere den bevorzugten Bedingungen nicht nachvollzogen werden. Wie die Vergleichsbeispiele 10 und 11 zeigen, konnte nur eine mäßige Aktivität und damit ein nur geringer Umsatz erzielt werden. Die umgesetzte HFPO-Masse findet sich zu etwa gleichen Teilen als Dimer ((I), n = 1) und - in der Regel unerwünschtes - Monomer ((I), n = 0) wieder.

Tertiäre Amine wie Triäthylamin oder N,N-Dimethylanilin selbst weisen unter autogenem Druck eine sehr geringe Oligomerisationsaktivität auf (DE-C 1 645 115), nach einer anderen Veröffentlichung sind sie sogar ausdrücklich als inaktiv beschrieben (EP-A 0 203 466). Erst ein Zweikomponenten-System aus physiologisch nicht unbedenklichen N,N-Dialkylanilin-Derivaten oder Pyridin-Derivaten mit Tetramethylharnstoff führt zu aktiven Katalysatoren für die Dimerisierung von HFPO.

Verkappte Harnstoffderivate, wie N,N,N',N'-tetrasubstituiertes Diaminodifluormethan, können auch zur Herstellung perfluorierter Carbonsäurefluoride aus Hexafluorpropenoxid als Katalysator eingesetzt werden (GB-A-1 550 268). Sie haben jedoch den Nachteil, daß der Temperaturbereich bei der Herstellung der Oligomeren und die Reaktionszeiten technisch unvorteilhaft sind.

Es bestand daher die Aufgabe, die Nachteile bei der katalytischen Oligomerisierung von HFPO auszuschließen und das Verfahren zu verbessern. Die Aufgabe wurde dadurch gelöst, daß tertiäre Diamine der Formel

$R^1R^2N\text{-}R\text{-}NR^3R^4$ (II)

in einem aprotischen polaren Lösemittel eine hohe katalytische Aktivität in der Oligomerisation von HFPO zeigen. Dieses System kann durch gezielten Zusatz von protonenaktiven Verbindungen selektiv die Dimerisierung von HFPO katalysieren.

Gegenstand der Erfindung ist ein Verfahren zur Herstellung perfluorierter Carbonsäurefluoride der allgemeinen Formel

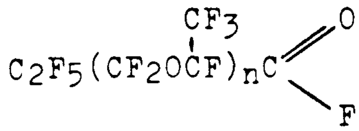 (I)

in der n gleich Null oder eine ganze Zahl von 1 bis 8, vorzugsweise 1 bis 5 ist, durch katalysierte Oligomerisation von Hexafluorpropenoxid, bei dem Hexafluorpropenoxid in einem aprotischen polaren Lösemittel in Gegenwart eines tertiären Diamins der allgemeinen Formel

$R^1R^2N\text{-}R\text{-}NR^3R^4$ (II)

in der R einen unverzweigten oder verzweigten, gesättigten oder ungesättigten Kohlenwasserstoffrest mit 1 bis 12 C-Atomen darstellt, der gegebenfalls Stickstoff oder Sauerstoff als Heteroatome enthaltende inerte Heterosubstituenten oder ein bezüglich der Reaktionsbedingungen inertes Heterokettenglied enthält, $R^1$ bis $R^4$ unabhängig voneinander cyclische oder acyclische, gesättigte oder ungesättigte Kohlenwasserstoffreste mit 1 bis 12 C-Atomen sind, die gegebenenfalls jeweils mindestens einen bezüglich der Reaktionskomponenten inerten Heterosubstituenten oder ein Heterokettenglied enthalten, wobei gegebenenfalls jeweils zwei der Reste $R^1$ bis $R^4$ zusätzlich über einen Kohlenwasserstoffrest oder mindestens ein Heteroatom verknüpft sind, und in Abwesenheit oder Gegenwart mindestens einer protonenaktiven Verbindung oligomerisiert wird. Durch Zusatz protonenaktiver Verbindungen wird eine Verschiebung der Produktselektivitäten zugunsten der kürzerkettigen Oligomeren (I) (n = 0, 1, 2, 3), insbesondere des Dimeren (I) (n = 1), bewirkt.

Heterosubstituenten, Heteroatome oder Heterokettenglieder sind Gruppierungen, die im allgemeinen Stickstoff oder Sauerstofff als Heteroatome enthalten.

Im allgeneinen arbeitet man so, daß aunüchst ein Diamin in einem aprotischen polaren Lösemittel dispergiert und dann - gegebenenfalls nach Zusatz einer protonenaktiven Verbindung - unter Rühren und Kühlung HFPO so eingegast wird, daß ein gewünschter Betriebsdruck nicht überschritten wird. Nach Abschluß der Reaktion wird die Produktphase abgetrennt und analysiert.

Tertiäre Diamine der allgemeinen Formel (II) sind leicht zugänglich. /J. Amer. Chem. Soc. (1957) 73, 3518; J. Org. Chem. (1987) 52, 467/ und teilweise kommerziell erwerblich.

Beispiele für Diamine (II) sind tertiäre, tetraalkylsubstituierte aliphatische Diaminverbindungen mit bis zu 10 C-Atomen in der Alkandiylkette und bis zu 6 C-Atomen in der Alkylgruppe sowie heterocyclische Diamine, vorzugsweise

N,N,N′,N′-Tetramethyl- und -ethylmethylendiamin,
N,N,N′,N′-Tetramethyl-, -ethyl- und -propylethylendiamin,
N,N,N′,N′-Tetramethylpropylen- und -isopropylendiamin,
N,N,N′,N′-Tetramethylhexylidendiamin sowie
Bis(3-methylpiperidino)-methan und N,N′-Dimethylpiperazin.

Als Verdünnungsmittel dienen aprotische polare Lösungsmittel wie aliphatische und aromatische Nitrile mit 2 bis 8 C-Atomen, aliphatische Dinitrile mit 5-8 C-Atomen und Ether der Formel $R'\text{-}(O\text{-}CH_2CHR'')_x\text{-}OR'$ in der R′ eine Alkylgruppe mit 1 bis 4 C-Atomen, R″ = Wasserstoff oder $CH_3$ und x eine ganze Zahl von 1 bis 6 bedeuten sowie cyclische Ether. Bevorzugt werden Nitrile oder Dinitrile wie Acetonitril, Propionitril, Butyronitril, Benzonitril oder Adiponitril sowie Ether wie Tetrahydrofuran, Ethylen- und Propylenglykoldialkylether und dessen höhere Oligomere oder Mischungen derselben. Die aprotischen polaren Lösemittel können durch inerte apolare Lösemittel wie Hexan verdünnt werden, ohne daß dies einen spürbaren Einfluß auf die Reaktion zeigt.

Die Menge des eingesetzten Lösungsmittels ist wenig kritisch.

Die Menge des eingesetzten Diamins (II) liegt zwischen 0,01 und 30 Mol-%, vorzugsweise zwischen 0,1 und 20 Mol-%, bezogen auf die eingesetzte HFPO-Menge. Das Katalysatorsystem kann - nach Abtrennung der schweren Produktphase - wieder verwendet werden; dabei tritt jedoch eine Selektivitätsverschiebung zugunsten der kürzerkettigen Oligomeren auf.

Das im erfindungsgemäßen Verfahren verwendete Katalysatorsystem stellt einen sehr aktiven Oligomerisationskatalysator dar; je nach eingehaltenem Druck und der Temperatur können Aktivitäten von größer 330 mol $mol^{-1}h^{-1}$ erreicht werden.

Die Oligomerisation ist bei Temperaturen von minus 30 bis plus 50 °C, vorzugsweise 5 bis 35 °C durchführbar. Niedrige Temperaturen führen zu einer Selektivitätsverschiebung zugunsten der höheren Oligomeren (n = 2, 3, ...) und bewirken einen geringeren HFPO-Umsatz.

Das Katalysator-System ist schon bei Normaldruck aktiv; ein erhöhter Druck im Reaktionsgefäß wird allerdings im Sinne eines schnelleren Umsatzes bevorzugt. Der Druck im Reaktionsgefäß kann über die Anströmgeschwindigkeit des gasförmigen oder flüssigen HFPO oder deren Mischungen beeinflußt werden.

Als protonenaktive Verbindungen können Substanzen der allgemeinen Formel

$R^5XH$ (III) und $R^6R^7R^8N$ (IV)

allein oder in Mischung eingesetzt werden. In den Formeln bedeutet: X Sauerstoff oder Schwefel; $R^5$ Wasserstoff, Alkyl-, Cycloalkyl-, Aryl-, Mono- oder Polyhydroxialkyl-, oder -Cycloalkyl, Mono- oder Polyhydroxiaryl-, Mono- oder Polyaminoalkyl- oder -cycloalkyl- Mono- oder Polyaminoaryl-, Mono- oder Poly(alkylamino)alkyl- oder -cycloalkyl- oder Mono- oder Poly(alkylamino)aryl-Rest; $R^6$ Wasserstoff, Mono- oder Polyhydroxialkyl- oder -cycloalkyl-, Mono- oder Polyhydroxiaryl-, Mono- oder Polyaminoalkyl- oder -cycloalkyl-, Mono- oder Polyalkylaminoaryl-, Mono- oder poly(alkylamino)alkyl- oder -cycloalkyl oder Mono- oder Poly(alkylamino)aryl-Rest; $R^7$ und $R^8$ sind gleich oder verschieden und entsprechen $R^6$ oder einem Alkyl-, Cycloalkyl-, oder Arylrest. $R^6$, $R^7$ und $R^8$ können verknüpft sein über eine Kohlenstoffkette mit oder ohne Heterokettenglieder oder über Heterosubstituenten.

Bevorzugt werden einfache protonenaktive Zusätze wie Wasser, Methanol, Glykol, Ammoniak, Diethylamin oder ähnliche.

Der Gehalt an protonenaktiven Verbindungen im verwendeten System ist kritisch bezüglich der Oligomerenverteilung. Wie Tabelle 1 am Beispiel des Systems N,N,N',N'-Tetramethylethylendiamin/Acetonitril/Wasser darstellt, tritt mit zunehmendem Gehalt des Katalysatorsystems an protonenaktiven Verbindungen eine Selektivitätsverschiebung zugunsten der kürzerkettigen Oligomeren, insbesondere auch des Dimeren, auf. Das Mengenverhältnis der protonenaktiven Substanz zum Diamin (II) hängt von der Anzahl der aktiven Protonen ab. Je nach angestrebtem Dimer-Gehalt kann in Gegenwart von bis zu 160 Mol-%, vorzugsweise bis zu 140 Mol-% aktiver Protonen, bezogen auf das eingesetzte Diamin (II) gearbeitet werden. In dieser und den folgenden Tabellen bedeutet "3+" den Anteil von Oligomeren mit n = 3 und höher, beispielsweise bis n = 8.

Tabelle 1 Selektivitäten in Masse-% der HFPO-Oligomeren (I)* in Abhängigkeit vom Wassergehalt des Katalysator-Systems $(CH_3)_2NCH_2CH_2N(CH_3)_2/CH_3CN$ (20 - 25°; 0,08 bar) in Mol-% bezogen auf die Diaminmenge

| $H_2O$ Mol-% | - | 11 | 22 | 36 | 50 | 64 |
|---|---|---|---|---|---|---|
| n=0 | 0,4 | 0,8 | 2,5 | 4,9 | 6,6 | 9,4 |
| n=1 | 27,4 | 54,5 | 75,2 | 80,5 | 86,6 | 85,4 |
| n=2 | 37,7 | 37,2 | 20,6 | 13,9 | 5,9 | 5,1 |
| n=3+ | 34,5 | 7,5 | 1,7 | 0,7 | 1,0 | 0,1 |

*) analysiert als Methylester

Der Vorteil des in dem Verfahren gemäß der Erfindung verwendeten Katalysators besteht darin, daß dieser zum einen die oben beschriebenen, mit dem Stand der Technik verbundenen Schwierigkeiten behebt, zum anderen aber ein auf der Basis einfacher, leicht handhabbarer und preiswert zugänglicher Chemikalien dem Stand derTechnik bezüglich der Oligomerisationsaktivität weit überlegenes System

darstellt. Vorteilhaft ist weiterhin die Flexibilität des Verfahrens, da mit ein und derselben Katalysatorvorstufe einerseits ein aktives katalytisches System für die Bildung höherer HFPO-Oligomere (n = 3-5) und andererseits nach Zusatz einer definierten Menge z.B. des denkbar einfachen Co-Katalysators Wasser ein sehr aktives System für die selektive Dimerisierung von HFPO erreicht wird. Vorteilhaft ist schließlich die hohe Lebensdauer des Katalysators verbunden mit einer sehr geringen Löslichkeit der Katalysatorspecies in der Produktphase, die es erlaubt, das Katalysatorsystem mehrmals einzusetzen. Die zur Reaktion verwendeten Apparaturen sowie die eingesetzten Substanzen sollten weitgehend wasserfrei sein, um eine Hydrolyse auszuschließen bzw. bei Zugabe von Wasser oder anderer protonenaktiven Verbindungen genaue Zahlenwerte einzuhalten.

Höhere HFPO-Oligomere, insbesondere das Trimere und Tetramere des HFPO, finden z.B. als Bausteine für perfluorierte Inertflüssigkeiten Verwendung. Das Dimere des HFPO dient u.a. als Zwischenprodukt bei der Herstellung von Perfluorpropylvinylether.

Beispiele

1) In einem Reaktionsgefäß, versehen mit einem Rührer, Innenthermometer und Manometer wurde unter Schutzgas 7,55 ml trockenes N,N,N',N'-Tetramethylethylendiamin und 50 ml trockenes Acetonitril vermischt. Nach Spülen des Gasraums mit HFPO wurde unter heftigem Rühren bei 25 - 30 °C gasförmiges HFPO so schnell eingeleitet, daß ein Betriebsüberdruck von 2 bar nicht überschritten wird. Nach 70 Minuten wurde die Produktphase (110 g) abgetrennt und in Form der Methylester gaschromatographisch analysiert. Bei quantitativem Umsatz wurde folgende Oligomerenverteilung (S = Selektivität in Masse-%) gefunden:

$$\begin{array}{l} \qquad\qquad\quad CF_3 \\ \qquad\qquad\quad | \\ C_2F_5(CF_2OCF)_nCOF \end{array}$$

(analysiert als Methylester)

| n | 1 | 2 | 3 | 4 | 5 |
|---|---|---|---|---|---|
| S (Masse-%) | 3,7 | 15,3 | 37,4 | 37,0 | 6,6 |

2) In einem Reaktionsgefäß, versehen mit Rührer, Innenthermometer, Manometer und einem kühlbaren Rückflußkühler, wurden 7,55 ml trockenes N,N,N',N'Tetramethylethylendiamin und 50 ml trockenes Acetonitril vermischt. Nach Spülen des Gasraums mit HFPO wird unter Rühren bei 25 - 30 °C gasförmiges HFPO so schnell eingeleitet, daß ein Überdruck von 0,08 bar nicht überschritten wurde. Nach 4 Stunden wurde die Produktphase (95 g) abgetrennt und in Form der Methylester gaschromatographisch analysiert. Bei quantitativem Umsatz wurde folgende Oligomerenverteilung (in Masse-%) bestimmt:

$$\begin{array}{l} \qquad\qquad\quad CF_3 \\ \qquad\qquad\quad | \\ C_2F_5(CF_2OCF)_nCOF \end{array}$$

(analysiert als Methylester)

| n | 0 | 1 | 2 | 3+ |
|---|---|---|---|---|
| S (Masse-%) | 0,4 | 27,4 | 37,7 | 34,5 |

3) In der in Beispiel 1 beschriebenen Apparatur wurden 7,55 ml trockenes N,N,N',N'-Tetramethylethylendiamin, 50 ml trockenes Acetonitril und Wasser gemischt. Nach Spülen des Gasraums mit HFPO wird unter Rühren bei 20 - 25 °C gasförmiges HFPO so schnell eingeleitet, daß ein Betriebsüberdruck von 2 bar nicht überschritten wurde. Nach 45 Minuten wurde die Produktphase (103 g) abgetrennt und in Form der Methylester gaschromatographisch analysiert. Bei quantitativem Umsatz wurde folgende Oligomerenverteilung (in Masse-%) gefunden:

$$C_2F_5(CF_2O\overset{\overset{CF_3}{|}}{C}F)_nCOF$$

(analysiert als Methylester)

| n | 0 | 1 | 2 | 3+ |
|---|---|---|---|---|
| S (Masse-%) | 2,0 | 74,2 | 22,1 | 1,7 |

4) In der in Beispiel 2 beschriebenen Apparatur wurden 7,55 ml trockenes N,N,N',N'-Tetramethylethylendiamin, 50 ml trockenes Acetonitril und 196 µl Wasser vermischt. Nach Spülen des Gasraums mit HFPO wird unter Rühren bei 20 - 25 °C gasförmiges HFPO so schnell eindosiert, daß ein Betriebsüberdruck von 0,08 bar nicht überschritten wurde. Nach 100 Minuten wurde die Produktphase (96 g) abgetrennt und in Form der Methylester gaschromatographisch analysiert. Bei quantitativem Umsatz wurde folgende Oligomerenverteilung (in Masse-%) bestimmt:

$$C_2F_5(CF_2O\overset{\overset{CF_3}{|}}{C}F)_nCOF$$

(analysiert als Methylester)

| n | 0 | 1 | 2 | 3+ |
|---|---|---|---|---|
| S (Masse-%) | 2,5 | 75,2 | 20,6 | 1,7 |

5) - 9) Entsprechend dem Beispiel 4 wurden verschiedene Katalysatorsysteme, bestehend aus 0,05 mol trockenem Diamin, 50 ml trockenem Acetonitril und 196 µl Wasser eingesetzt. Bei quantitativem Umsatz wurden die in Tabelle 2 aufgeführten Oligomerenverteilungen gefunden.

Tabelle 2

| Selektivitäten in Masse-% der HFPO-Oligomeren (I)* für verschiedene Diamine (Lösemittel: Acetonitril 196 µl Wasser) | | | | | |
|---|---|---|---|---|---|
| Beispiel | Diamin | S (Masse-%) | | | |
| | | n = 0 | n = 1 | n = 2 | n = 3+ |
| 5 | N,N,N',N'-Tetramethylmethylendiamin | 4,4 | 80,9 | 13,8 | 0,8 |
| 6 | N,N,N',N'-Tetraethylethylendiamin | 5,0 | 83,2 | 11,1 | 0,7 |
| 7 | N,N,N',N'-Tetramethyl 1,6-hexandiamin | 2,3 | 70,3 | 24,7 | 2,6 |
| 8 | N,N'-Dimethyl-1,4-piperazin | 3,2 | 82,2 | 14,1 | 0,6 |
| 9 | Bis-(3-methyl)-piperidinomethan | 2,9 | 76,8 | 17,6 | 2,6 |

*) analysiert als Methylester

Vergleichsbeispiel 10)

In der in Beispiel 2 beschriebenen Apparatur wurden 7 ml trockenes Triethylamin, 50 ml trockenes Acetonitril und 26 µl Wasser vermischt. Nach Spülen des Gasraums mit HFPO wurde unter Rühren HFPO so eingegast, daß ein Betriebsdruck von 0,08 bar nicht überschritten wurde. Während der Einleitung bei 20 °C Umgehungstemperatur fällt die Innentemperatur auf minus 25 °C. Nach ca. 135 Minuten wurde die Produktphase (91 g) abgetrennt und in Form der Methylester gaschromatographisch analysiert. Bei einem Umsatz von ca. 55 % wurde folgende Oligomerenverteilung (in Masse-%) bestimmt:

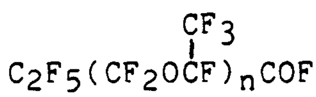

(analysiert als Methylester)

| n | 0 | 1 | 2 |
|---|---|---|---|
| S (Masse-%) | 50,9 | 49,1 | - |

Vergleichsbeispiel 11)

Es wurde wie in Beispiel 10 gearbeitet, aber die Reaktion bei minus 20 °C begonnen. Im Verlauf des Einleitens von HFPO fällt die Temperatur auf minus 24 °, obwohl das Reaktionsgefäß von außen erwärmt wurde. Nach 140 Minuten wurde die Produktphase (91 g) abgetrennt und in Form der Methylester gaschromatographisch analysiert. Bei einem Umsatz von ca. 20 % wurde folgende Oligomerenverteilung (in Masse-%) bestimmt.

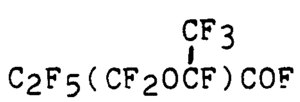

(analysiert als Methylester)

| n | 0 | 1 | 2 |
|---|---|---|---|
| S (Masse-%) | 50 | 50 | - |

12) Entsprechend dem Beispiel 4 wurde ein Katalysator-System aus 7,55 ml trockenem N,N,N',N'-Tetramethylethylendiamin, 50 ml trockenem Acetonitril und 882 µl trockenem Methanol für die HFPO-Oligomerisation eingesetzt. Nach 150 Minuten wurde die Produktphase (113 g) abgetrennt und in Form der Methylester gaschromatographisch analysiert. Bei vollständigem Umsatz ergab sich folgende Oligomerenverteilung:

$$C_2F_5(CF_2O\overset{CF_3}{\overset{|}{C}}F)_nCOF$$

(analysiert als Methylester)

| n | 0 | 1 | 2 | 3+ |
|---|---|---|---|---|
| S (Masse-%) | 2,3 | 70,6 | 24,5 | 2,6 |

13) Entsprechend dem Beispiel 4 wurde ein Katalysatorsystem aus 7,55 ml trockenem N,N,N',N'-Tetramethylethylendiamin, 50ml trockenem Acetonitril und 1 ml trockenem Anilin für die HFPO-Oligomerisation eingesetzt. Bei vollständigem Umsatz wurde für die Produktphase (89 g) folgende Oligomeren-Verteilung (in Masse-%) ermittelt:

$$C_2F_5(CF_2O\overset{CF_3}{\overset{|}{C}}F)_nCOF$$

(analysiert als Methylester)

| n | 0 | 1 | 2 | 3+ |
|---|---|---|---|---|
| S (Masse-%) | 2,3 | 69,5 | 25,1 | 3,1 |

14) Entsprechend dem Beispiel 3 wurde ein Katalysator-System aus 0,31 ml trockenem N,N,N',N'-Tetramethylethylendiamin und 50 ml trockenem Acetonitril für die HFPO-Oligomerisation eingesetzt. Nach 40 Minuten wurde die Produktphase (94 g) abgetrennt und in Form der Methylester gaschromatographisch analysiert. Bei quantitativem Umsatz ergab sich folgende Oligomerenverteilung:

$$C_2F_5(CF_2O\overset{CF_3}{\overset{|}{C}}F)_nCOF$$

(analysiert als Methylester)

| n | 0 | 1 | 2 | 3+ |
|---|---|---|---|---|
| S (Masse-%) | 0,5 | 30,2 | 45,2 | 3,6 |

15) Beispiel 4 wurde wiederholt unter Ersatz des Acetonitrils durch Tetrahydrofuran. Nach 140 Minuten wurde die Produktphase (86 g) abgetrennt und in Form der Methylester gaschromatographisch analysiert. Bei 99 %igem Umsatz wurde folgende Oligomerenverteilung (in Masse-%) bestimmt:

$$C_2F_5(CF_2O\overset{CF_3}{CF})_nCOF$$

(analysiert als Methylester)

| n | 0 | 1 | 2 | 3+ |
|---|---|---|---|---|
| S (Masse-%) | 4,1 | 60,3 | 28,0 | 7,6 |

16) In einem Reaktionsgefäß, versehen mit Rührer, Innenthermometer, Manometer und einem kühlbaren Rückflußkühler wurden 65,4 ml trockenes N,N,N',$N_1$-Tetramethylethylendiamin, 433 ml trockenes Acetonitril und 1,39 ml Wasser vermischt. Nach dem Spülen des Gasraums mit HFPO wurde unter Rühren bei 20 - 25 °C gasförmiges HFPO so schnell eingeleitet, daß ein Überdruck von 0,08 bar nicht überschritten wurde. Nach Beendigung der Reaktion wurde die Produktphase abgetrennt und dann erneut HFPO eingeleitet. Dieser Vorgang kann mehrmals wiederholt werden. Tabelle 3 gibt exemplarisch die Oligomerenverteilung verschiedener, mit demselben Katalysatorgemisch hergestellter Oligomerchargen wieder.

Tabelle 3

| Selektivitäten in Masse-% der HFPO-Oligomeren (I)* in verschiedenen, mit demselben Katalysatorsystem hergestellten Chargen. | | | | | | |
|---|---|---|---|---|---|---|
| Charge | m kg | m (gesamt) kg | Selekt. in Masse-% | | | |
| | | | n = 0 | n = 1 | n = 2 | n = 3 |
| 1 | 0,46 | 0,46 | 2,0 | 74,9 | 21,0 | 2,1 |
| 4 | 0,32 | 1,43 | 3,4 | 81,6 | 14,2 | 0,8 |
| 6 | 0,45 | 2,39 | 3,8 | 83,7 | 11,7 | 0,8 |

*) Analysiert als Methylester
m = Masse der Oligomeren (I) der jeweiligen Charge

## Patentansprüche

**1.** Verfahren zur Herstellung perfluorierter Carbonsäurefluoride der allgemeinen Formel

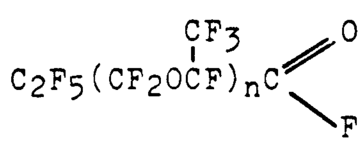

(I)

in der n gleich Null oder eine ganze Zahl von 1 bis 8 ist, durch katalysierte Oligomerisation von Hexafluorpropenoxid dadurch gekennzeichnet, daß Hexafluorpropenoxid in einem aprotischen polaren Lösemittel in Gegenwart eines tertiären Diamins der allgemeinen Formel

$R^1R^2N\text{-}R\text{-}NR^3R^4$ (II)

in der
R einen unverzweigten oder verzweigten, gesättigten oder ungesättigten Kohlenwasserstoffrest mit 1

bis 12 C-Atomen darstellt, der gegebenfalls Stickstoff oder Sauerstoff als Heteroatome enthaltende inerte Heterosubstituenten oder einbezüglich der Reaktionsbedingungen inertes Heterokettenglied enthält,
$R^1$ bis $R^4$ unabhängig voneinander cyclische oder acyclische, gesättigte oder ungesättigte Kohlenwasserstoffreste mit 1 bis 12 C-Atomen sind, die gegebenenfalls jeweils mindestens einen bezüglich der Reaktionskomponenten inerten Heterosubstituenten oder ein Heterokettenglied enthalten, wobei gegebenenfalls jeweils zwei der Reste $R^1$ bis $R^4$ zusätzlich über einen Kohlenwasserstoffrest oder mindestens ein Heteroatom verknüpft sind, und in Abwesenheit oder Gegenwart mindestens einer protonenaktiven Verbindung oligomerisiert wird.

**2.** Verfahren nach Anspruch 1, dadurch gekennzeichnet, daß mindestens eine protonenaktive Verbindung der allgemeinen Formeln

$R^5XH$ (III) und $R^6R^7R^8N$ (IV)

verwendet wird, wobei in den Formeln bedeuten
X Sauerstoff oder Schwefel;
$R^5$ Wasserstoff, Alkyl-, Cycloalkyl-, Aryl-, Mono- oder Polyhydroxialkyl-, oder -Cycloalkyl, Mono- oder Polyhydroxiaryl-, Mono- oder Polyaminoalkyl- oder -cycloalkyl-Mono- oder Polyaminoaryl-, Mono- oder Poly(alkylamino)alkyl-oder -cycloalkyl- oder Mono- oder Poly(alkylamino)aryl-Rest;
$R^6$ Wasserstoff, Mono- oder Polyhydroxialkyl- oder -cycloalkyl-, Mono- oder Polyhydroxiaryl-, Mono- oder Polyaminoalkyl- oder -cycloalkyl-, Mono- oder Polyalkylaminoaryl-, Mono- oder Poly(alkylamino)-alkyl- oder -cycloalkyl oder Mono- oder Poly(alkylamino)aryl-Rest;
$R^7$ und $R^8$ sind gleich oder verschieden und entsprechen $R^6$ oder einem Alkyl-, Cycloalkyl-, oder Arylrest; gegebenenfalls sind $R^6$, $R^7$ und $R^8$ über eine Kohlenstoffkette mit oder ohne Heterokettengliedern verknüpft.

**3.** Verfahren nach Anspruch 1, dadurch gekennzeichnet, daß ohne Zusatz einer protonenaktiven Verbindung gearbeitet wird.

**4.** Verfahren nach den Ansprüchen 1 oder 2, dadurch gekennzeichnet, daß in Gegenwart von 1 bis 140 Mol-% protonenaktiver Verbindung, bezogen auf das eingesetzte Diamin, gearbeitet wird.

**5.** Verfahren nach einem oder mehreren der Ansprüche 1, 2 und 4, dadurch gekennzeichnet, daß als protonenaktive Verbindung Wasser, Methanol, Glykol, Ammoniak oder Diethylamin verwendet wird.

**6.** Verfahren nach einem oder mehreren der Ansprüche 1 bis 5, dadurch gekennzeichnet, daß als aprotisches, polares Lösungsmittel ein aliphatisches oder aromatisches Nitril mit 2 bis 8 Kohlenstoffatomen, ein aliphatisches Dinitril mit 5 bis 8 Kohlenstoffatomen, ein Ether der Formel

$R'\text{-}(O\text{-}CH_2CHR'')_xOR'$

in der R′ eine Alkylgruppe mit 1 bis 4 C-Atomen,
R″ = Wasserstoff oder $CH_3$ und x = eine ganze Zahl von 1 bis 6 bedeuten oder ein cyclischer Ether verwendet wird.

**7.** Verfahren nach einem oder mehreren der Ansprüche 1 bis 6, dadurch gekennzeichnet, daß die Oligomerisierung bei minus 30 bis plus 50 °C durchgeführt wird.

**8.** Verfahren nach einem oder mehreren der Ansprüche 1 bis 7, dadurch gekennzeichnet, daß die Menge des eingesetzten Diamins (II) 0,01 bis 30 Mol.-%, bezogen auf das eingesetzte HFPO, beträgt.

**9.** Verfahren nach einem oder mehreren der Ansprüche 1 bis 8, dadurch gekennzeichnet, daß als Heteroatom, Heterosubstituent oder Heterokettenglied Sauerstoff oder Stickstoff vorhanden ist.

## Claims

**1.** A process for the preparation of perfluorinated carbonyl fluorides of the formula

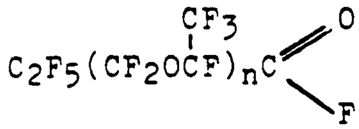

(I)

in which n is equal to zero or an integer from 1 to 8, by catalyzed oligomerization of hexafluoropropene oxide, which comprises oligomerization of hexafluoropropene oxide in an aprotic polar solvent in the presence of a tertiary diamine of the formula

$R^1R^2N\text{-}R\text{-}NR^3R^4$ (II)

in which R represents an unbranched or branched, saturated or unsaturated hydrocarbon radical which has 1 to 12 carbon atoms and optionally contains inert hetero substituents containing nitrogen or oxygen as hetero atoms, or optionally contains a hetero chain member which is inert with respect to the reaction conditions, $R^1$ to $R^4$ are, independently of one another, cyclic or acyclic, saturated or unsaturated hydrocarbon radicals which have 1 to 12 carbon atoms and optionally each contain at least one hetero chain member or at least one hetero substituent which is inert toward the reaction components, with, in addition, any two of the radicals $R^1$ to $R^4$ optionally being linked via a hydrocarbon radical or at least one hetero atom, and in the absence or presence of at least one protonic compound.

**2.** The process as claimed in claim 1, wherein at least one protonic compound of the formulae

$R^5XH$ (III) and $R^6R^7R^8N$ (IV)

is used, where the meanings in the formulae are X oxygen or sulfur; $R^5$ hydrogen, alkyl, cycloalkyl, aryl, mono- or polyhydroxyalkyl or -cycloalkyl, mono- or polyhydroxyaryl, mono- or polyaminoalkyl or -cycloalkyl, mono- or polyaminoaryl, mono- or poly(alkylamino)alkyl or -cycloalkyl or mono- or poly-(alkylamino)aryl radical; $R^6$ hydrogen, mono- or polyhydroxyalkyl or -cycloalkyl, mono- or polyhydroxyaryl, mono- or polyaminoalkyl or -cycloalkyl, mono- or polyalkylaminoaryl, mono- or poly(alkylamino)-alkyl or -cycloalkyl or mono- or poly(alkylamino)aryl radical;
$R^7$ and $R^8$ are identical or different and correspond to $R^6$ or to an alkyl, cycloalkyl or aryl radical, and, where appropriate, $R^6$, $R^7$ and $R^8$ are linked via a carbon chain with or without hetero chain members.

**3.** The process as claimed in claim 1, wherein no protonic compound is added.

**4.** The process as claimed in claims 1 or 2, which is carried out in the presence of 1 to 140 mol-% of protonic compound relative to the diamine employed.

**5.** The process as claimed in one or more of claims 1, 2 and 4, wherein water, methanol, glycol, ammonia or diethylamine is used as protonic compound.

**6.** The process as claimed in one or more of claims 1 to 5, wherein an aliphatic or aromatic nitrile having 2 to 8 carbon atoms, an aliphatic dinitrile having 5 to 8 carbon atoms, an ether of the formula

$R'\text{-}(O\text{-}CH_2CHR'')_xOR'$

in which R' denotes an alkyl group having 1 to 4 carbon atoms, R'' denotes hydrogen or $CH_3$ and x denotes an integer from 1 to 6, or a cyclic ether, is used as aprotic polar solvent.

**7.** The process as claimed in one or more of claims 1 to 6, wherein the oligomerization is carried out at minus 30 to plus 50 °C.

**8.** The process as claimed in one or more of claims 1 to 7, wherein the amount of diamine (II) employed is 0.01 to 30 mol-% relative to the HFPO employed.

**9.** The process as claimed in one or more of claims 1 to 8, wherein oxygen or nitrogen is present as hetero atom, hetero substituent or hetero chain member.

**Revendications**

**1.** Procédé pour préparer des fluorures d'acides carboxyliques perfluorés de formule générale

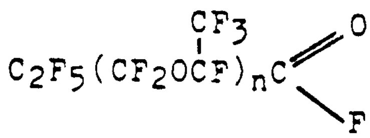

(I)

dans laquelle n vaut 0 ou est un nombre entier de 1 à 8, par oligomérisation catalysée d'oxyde d'hexafluoropropène, dans lequel on oligomérise de l'oxyde d'hexafluoropropène dans un solvant aprotique polaire, en présence d'une diamine tertiaire de formule générale

$R^1R^2N\text{-}R\text{-}NR^3R^4$ (II)

dans laquelle R est un radical hydrocarboné à chaîne droite ou ramifiée, éventuellement saturée, ayant de 1 à 12 atomes de carbone, qui éventuellement contient des hétéro-substituants inertes, contenant de l'azote ou de l'oxygène en tant qu'hétéro-atomes, ou un hétéro-chaînon inerte dans les conditions de la réaction,

$R^1$ à $R^4$, indépendamment les uns des autres, sont des radicaux hydrocarbonés cycliques ou acycliques, saturés ou insaturés, ayant de 1 à 12 atomes de carbone, qui éventuellement contiennent chacun au moins un hétérosubstituant inerte vis-à-vis des constituants de la réaction, ou un hétéro-chaînon, éventuellement deux des radicaux $R^1$ à $R^4$ étant en outre reliés l'un à l'autre par l'intermédiaire d'un résidu hydrocarboné ou d'au moins un héréro-atome, et en l'absence ou en présence d'au moins un composé à activité de proton.

**2.** Procédé selon la revendication 1, caractérisé en ce qu'on utilise au moins un composé à activité de proton de formule générale

$R^5XH$ (III) et $R^6R^7R^8N$ (IV)

où, dans les formules, X est un oxygène ou un soufre ; $R^5$ est un hydrogène ou un radical alkyle, cycloalkyle, aryle, mono- ou polyhydroxyalkyle ou -cycloalkyle, mono-ou polyhydroxyaryle, mono- ou polyaminoalkyle ou -cycloalkyle, mono- ou polyaminoaryle, mono- ou poly(alkylamino)alkyle ou -cycloalkyle, ou mono- ou poly(alkylamino)aryle ; $R^6$ est un hydrogène ou un radical mono- ou polyhydroxyalkyle ou -cycloalkyle, mono- ou polyhydroxyaryle, mono- ou polyaminoalkyle ou -cycloalkyle, mono-ou polyalkylaminoaryle, mono- ou poly(alkylamino)alkyle ou -cycloalkyle, ou mono- ou poly-(alkylamino)aryle ; $R^7$ et $R^8$ sont identiques ou différents et correspondent à $R^6$ ou à un radical alkyle, cycloalkyle ou aryle ; et éventuellement $R^6$, $R^7$ et $R^8$ sont reliés par l'intermédiaire d'une chaîne carbonée, avec ou sans hétêro-chaînons.

**3.** Procédé selon la revendication 1, en ce qu'on travaille sans ajouter de composés à activité de proton.

**4.** Procédé selon les revendications 1 ou 2, caractérisé en ce qu'on travaille en présence de 1 à 140 % en moles d'un composé à activité de proton, par rapport à la diamine utilisée.

**5.** Procédé selon l'une ou plusieurs des revendications 1, 2 et 4, caractérisé en ce qu'on utilise comme composé à activité de proton l'eau, le méthanol, le glycol, l'ammoniac ou la diéthylamine.

6. Procédé selon l'une ou plusieurs revendications 1 à 5, caractérisé en ce qu'on utilise comme solvant aprotique polaire un nitrile aliphatique ou aromatique ayant de 2 à 8 atomes de carbone, un dinitrile aliphatique ayant de 5 à 8 atomes de carbone, un éther de formule

$R'-(O-CH_2CHR'')_x-OR'$

où R' est un groupe alkyle ayant de 1 à 4 atomes de carbone, R'' est un hydrogène ou $CH_3$, et x est un nombre entier de 1 à 6, ou encore un éther cyclique.

7. Procédé selon l'une ou plusieurs des revendications 1 à 6, caractérisé en ce que l'oligomérisation est mise en oeuvre à une température de -30 à +50 °C.

8. Procédé selon l'une ou plusieurs des revendications 1 à 7, caractérisé en ce que la quantité de la diamine(II)utilisée est de 0,01 à 30 % en moles par rapport au HFPO utilisé.

9. Procédé selon l'une ou plusieurs des revendications 1 à 8, caractérisé en ce qu'on a comme hétéro-atome, hétéro-substituant ou hétéro-chaînon l'oxygène ou l'azote.